# EUROPEAN PATENT SPECIFICATION

(11) **EP 1 424 894 B1**
(45) Date of publication and mention of the grant of the patent: **12.11.2008**
(21) Application number: 02705724.9
(22) Date of filing: 11.01.2002
(51) Int. Cl.: A01N 43/04, A01N 63/00, A01N 65/00, A61K 31/70, A61K 48/00, A61P 35/00

(54) **INHIBITION OF PATHOLOGICAL ANGIOGENESIS IN VIVO**
HEMMUNG DER PATHOLOGISCHEN ANGIOGENESE IN VIVO
INHIBITION D'UNE ANGIOGENESE PATHOLOGIQUE IN VIVO

(30) Priority: 12.01.2001 US 261381 P
(43) Date of publication of application: 09.06.2004
(73) Proprietor: Sbarro Health Research Organization, Inc., Radnor, PA 19087 (US)
(72) Inventor: GIOVAN, Giacomo, Giordano, I-80122 Naples (IT)
(74) Representative: Vossius & Partner
(86) International application number: PCT/US2002/000668
(87) International publication number: WO 2002/054851

(56) References cited:
- EP-A- 0 848 063
- WO-A-00/24408
- WO-A-98/37185
- US-A- 5 532 340
- US-A- 5 807 681
- US-A- 5 840 506

## Description

### FIELD OF THE INVENTION

The present invention relates to use of vectors expressing pRb2/p130 for the preparation of pharmaceutical compositions for the inhibition of angiogenesis in a target area of a patient. Specifically, the present invention involves the down-regulation of an angiogentic factor expression in a target tissue by delivery of pRb2/p130 and induction of its expression for the inhibition of angiogenesis in the target tissue.

### BACKGROUND OF THE INVENTION

Angiogenesis is the formation of new blood vessels from preexisting ones. Angiogenesis is an essential step in the progression of tissue (e.g., tumor tissue) formation and development because tissue growth beyond a certain point depends on the supply of oxygen and nutrients from this vascular network. For example, with respect to tumor tissue, only tumors of 1-2 mm of diameter can receive all sufficient nutrients by diffusion; therefore, additional growth depends on the development of an adequate blood supply through angiogenesis. (Folkman, J. Nat'l Cancer Inst. 82:4-6, 1990).

Angiogenesis is driven by a balance between different positive and negative effector molecules influencing the growth rate of capillaries. Various angiogenetic and anti-angiogenetic factors have been cloned to date and are known (Leung et al., Science. 246: 1306-9,1989; Ueno et al., Biochem Biophys Acta. 1382: 17-22, 1998; Miyazono et al., Prog Growth Factor Res. 3: 207-17,1991). Vascular endothelial growth factor (VEGF) and trombospondin-1 (TSP-1) are two of the most well studied. VEGF is an angiogenic factor as opposed to TSP-1, which functions as an antiangiogenic molecule (Tuszynski et al., Bioessays. 18: 71-6,1996; Dameron, et a;. Science. 265: 1582-4, 1994). Normal vessel growth results by balanced and coordinated expression of these opposing factors. A switch from normal to uncontrolled vessel growth can occur by up-regulating angiogenesis stimulators or down-regulating angiogenesis inhibitors, suggesting that the angiogenetic process is tightly regulated by the oscillation between these opposing forces (Bouck et al., Adv Cancer Res. 69: 135-74,1996). For example, in tumor tissues the switch to an angiogenic phenotype occurs as a distinct step before progression to a neoplastic phenotype and is linked to epigenetic or genetic changes (Hanahan et al., Cell. 86: 353-64, 1996). In support of this theory, mRNA expression of VEGF is up-regulated in aggressive tumor cell lines expressing an activated *ras* oncogene (Rak et al., Neoplasia. 1: 23-30, 1999). Conversely, transcription of VEGF is down-regulated in these same tumor cell lines after disruption of the mutant *ras* allele, thus eliminating VEGF expression and rendering the cells incapable of tumor formation *in vivo.* (Stiegler et al., J Cell Physiol. 179: 233-6, 1999). The switch to an angiogenic phenotype has also been associated with the inactivation of the tumor suppressor gene p53 (Holmgren et al., Oncogene. 17: 819-24, 1998). Conversely, cell lines that are p16 deleted revert to an anti-angiogenic phenotype upon the restoration of wild type cyclin dependent kinase (cdk) inhibitor p16. Harada et al., Cancer Research. 59: 3783-3789, 1999.

Besides tumors, VEGF also has been reported to cause pathological angiogenesis and this contributes to conditions such as diabetic retinopathy, rheumatoid arthritis, choroidal neovascularization, syogenic granuloma, endometriosis, pulmonary edema, and pulmonary tuberculosis.

The retinoblastoma (RB) gene family includes three members: the Rb tumor suppressor *RB*/*p105,p107,* and *RB2*/*p130.* These proteins are highly homologous in the "pocket" region, composed of subdomains A and B separated by a spacer region that is highly conserved among each of the proteins (Lee et al., Science 235: 1394-9, 1987; Ewen et al., Cell 66: 1155-64, 1991; Mayol et al., Oncogene. 8: 2561-6, 1993; Li, et al , Genes Dev. 7: 2366-77, 1993; Hannon et al., Genes Dev. 7: 2378-91, 1993). This functional domain is targeted by viral oncoproteins and is responsible for many functional interactions (Stiegler et al., J Cell Biochem Suppl. 31: 30-6, 1998). Functionally, all the Rb family members show cell type specific growth suppressive properties unique to each member. They each bind and temporally modulate in a distinct manner the activity of specific members of the E2F family of transcription factors, and are regulated by phosphorylation in a cell cycle dependent-manner (Paggi et al., J Cell Biochem. 62: 418-30, 1996). The structural identities of these proteins underlie similar but distinct functional properties. In fact, all three family members inhibit cell-cycle progression in the G₁ phase of the cell cycle (Zhu et al., Genes Dev. 7: 1111-25, 1993; Claudio et al., Cancer Res. 56: 2003-8, 1996; Huang et al., Science. 242: 1563-6, 1988). Interestingly, the retinoblastoma family of proteins exhibit unique growth suppressive properties; although they may complement each other, their functions are not fully redundant (Claudio et al., Cancer Res. 54: 5556-60,1994).

In several tumor cell lines pRb2/p130 mediates a G₀/G₁ phase cell-cycle arrest including the human T98G glioblastoma cell line, which is resistant to the suppressive effects of both pRb/p105 and p107 (Zhu et al., Genes Dev. 7: 1111-25, 1993; Claudio et al., Cancer Res. 56: 2003-8,1996; Claudio et al., Cancer Res. 54: 5556-60, 1994). It has been shown in the present invention that by expressing RB2/p130, the fine tuned angiogenetic balance can be disrupted in tissues. More specifically, vascular endothelial growth factor (VEGF) protein (an angiogenic factor) expression both *in vitro* and *in vivo* can be down-regulated by expressing RB2/p130. It has also been shown here that the down-regulation of angiogenic factor vascular endothelial growth factor (VEGF) protein is sufficient to inhibit angiogenes in a tissue in vivo.

### SUMMARY OF THE INVENTION

The present invention provides uses of a vector expressing pRb2/p130 for the preparation of a pharmaceutical composition for the treatment of VEGF involved disease conditions such as certain tumors and cancers, diabetic retinopathy, choroidal neovascularization, rheumatoid arthritis, pyogenic granuloma, female reproductive cycling disorders.

In the present invention, it has been found that RB2/p130 can significantly decrease VEGF RNA and protein expression *in vitro* and *in vivo* in both rodent and human tissues sufficient to inhibit pathological angiogenesis. Additionally, enhanced RB2/p130 gene expression down-regulated the activity of the VEGF promoter in a tetracycline-regulated pRb2/p130 system.

In a general aspect of the invention, use of a vector expressing pRb2/p130 for the preparation of a pharmaceutical composition to prevent angiogenesis in a target tissue area of a patient in need of the prevention is provided. Target tissue area can be a tissue where angiogenesis is essential for its progression to cause certain undesirable disease or condition such as tumor formation. The use volves a vector expressing pRb2/p130 at levels sufficient to inhibit the formation of the angiogenesis in the target area, wherein the vector is an adenoviral vector or a retroviral vector. The use of the invention specifically modulates the expression of a gene of interest which encodes a protein, the expression of which is associated with angiogenesis within a patient. Contacting one or more cells, which express the gene, with a virus vector expressing Rb2/p130 or a fragment thereof at levels sufficient to specifically modulate the expression of the gene and thereby affect the level of the protein encoded by the gene of interest is envisaged

The gene of interest is VEGF or its homologues within the same gene family that are involved in angiogenesis. The Rb2/p130 or a fragment thereof interferes with promoter regulation of said VEGF or interferes with mRNA expression of the VEGF or may also interfere with protein expression of the VEGF. The fragment Rb2/p130 should be sufficient enough to bring about the down regulation of VEGF sufficient for the inhibition of angiogenesis.

Cells can be, for example, cells of a human tumor, cells of a human cancer, cells of a retinal tissue, cells of a retinal pigment epithelium, cells of a synovial tissue. A VEGF inhibiting peptide that is capable of specifically influencing VEGF expression and thereby exerting an inhibitory effect on angiogenesis in a tissue of a patient is also provided. The cancer that can be treated with this use includes human glioblastoma, melanoma, breast cancer, prostate cancer, colon cancer, blood cancer, osteosarcoma, lung cancer, endometrial cancer and stomach carcinoma.

The vector used is either viral vector or a plasmid vector. Among viral vector a retroviral vector or an adenoviral vector can be used to deliver and express pRb2/p130 in the target tissue to bring about the down-regulation of VEGF in the target area

In yet another aspect of the invention a use of a recombinant vector expressing pRb2/p130 for the preparation of a pharmaceutical composition to prevent angiogenesis in a cancer tissue of a patient to treat cancer is provided. This use includes said recombinant vector expressing pRb2/p130 at levels sufficient to inhibit the formation of the angiogenesis in the cancer tissue. The recombinant vector used can be an adenoviral vector or a retroviral vector or any other suitable vector.

In a further aspect of the invention a use of a recombinant vector expressing pRb2/p130 for the preparation of a pharmaceutical composition for inhibiting angiogenesis in lung cancer tissue of a patient, the use comprising said recombinant vector expressing pRb2/p130 at levels sufficient to down-regulate VEGF expression so as to inhibit angiogenesis in the tissue, wherein the vector is an adenoviral vector or a retroviral vector.

In yet another aspect of the invention a use of a recombinant vector expressing pRb2/p130 for the preparation of a pharmaceutical composition to treat rheumatoid arthritis in a patient is provided. The use involves said recombinant vector expressing pRb2/p130 at levels sufficient to down-regulate VEGF expression in synovial tissue and inhibit angiogenesis in the synovial tissue.

In yet another aspect of the invention a use of a recombinant vector expressing pRb2/p130 for the preparation of a pharmaceutical composition to treat diabetic retinopathy in a patient is provided. This use involves said recombinant vector expressing pRb2/p130 at levels sufficient to down-regulate VEGF expression in the retina and inhibit angiogenesis in the retina.

In yet another aspect of the invention a use of a recombinant vector expressing pRb2/p130 for the preparation of a pharmaceutical composition to treat choroidal neovascularization in a patient is provided. This use involves said recombinant vector expressing pRb2/p130 at levels sufficient to down-regulate VEGF expression in said tissue and inhibit angiogenesis in the choroidal tissue.

### BRIEF DESCRIPTION OF THE FIGURES

**Figure 1** shows Northern blot analysis of H23 cells transduced with either Ad-CMV or Ad-*RB2*/*p130.*
**Figure 2** is a bar graph illustrating VEGF luciferase activity in HJC#12 cells VEGF and E2F promoter luciferase constructs were transiently transfected into HJC#12 cells and subsequently pRb2/p130 expression was induced (-Tet). The promoters used are indicated on the top.
**Figure 3** is a Graphic representation of a single experiment of a VEGF Enzyme-Linked-Immunosorbent-Assay (ELISA) in the conditioned medium of H23 and HJC#12 cells following *RB2*/*p130* over expression.
**Figure 4** shows Western blot analysis of VEGF protein abundance upon pRb2/p130 enhanced expression in H23 and HJC#12 cells, Cell lines are indicated on the top.
**Figure 5** shows Immunohistochemical analysis of VEGF and CD31 of HJC Δ5 and HJC#12 tumor grafts grown in *nude* mice.
**Figure 6** shows Immunohistochemical analysis of VEGF and CD31 of H23 tumor grafts grown in *nude* mice.

### DETAILED DESCRIPTTON OF THE INVENTION

The present invention relates to the use of vectors expressing pRb2/130 for the preparation of pharmaceutical compositions for inhibiting angiogenesis. It is shown in the present invention that *RB2*/*p130* modulates angiogenic factor (VEGF) expression and inhibits angiogenesis *in vivo.* Down-regulation of VEGF (an agniogenic factor) expression in tumor cells and inhibition of angiogenesis in tumor tissues are used as examples to illustrate the present invention. Down-regulation of VEGF expression in both *in vitro* and in *vivo* followed enhanced RB2/p130 expression is shown in the present invention.

Tumors secrete angiogenic factors such as the vascular permeability factor/vascular endothelial growth factor (VPF/VEGF) which interact with specific receptors on the surface of vascular endothelial cells to enhance angiogenesis.

Tumorigenesis is a multistep process that involves several genetic changes resulting in uncontrolled cellular proliferation and inhibition of apoptosis (Vogelstein. et al, Trends Genet. 9: 138-41, 1993). Tumor growth and cellular proliferation are linked by the tumor's ability to foster proper vascularization from the host to the alien tumor graft. Recent evidence shows that tumors do not grow larger than a few millimeters in size unless vascularized by the host (Folkman, J. The molecular basis of cancer. In: J. Mendelson, P. M. Howley, M. A. Israle, and L. A. Liotta (eds.), pp. 206-232. Philadelphia: W.B. Saunders, 1995). Tissue progression (e.g. tumor progression) and growth require an appropriate rate of blood vessel formation related to the rate of neoplastic cellular proliferation, otherwise tumor necrosis and eventual calcification would result.

pRB2/p130 codes for a 130kDa protein, which seems to be more restricted in its function to control gene expression in cells that are not in the proliferative cell cycle(Nevins, 1998). This protein is stabilized and activated as soon as the cell withdraws from the cell cycle during cell cycle arrest and cellular differentiation. pRB2/p130 is powerful in arresting cell culture models if ectopically expressed (Claudio et al., 1996; Claudio et al., 1994 Cancer Res, 54, 5556-60). Even though all three members (pRB/p105, pRB2/p130 and pRBL1/p107) share high homologies and overlapping features and activities, each protein has a unique function and plays a unique, nonredundant role (Claudio et al., 1994; Mulligan and Jacks, 1998; Mulligan et al., 1998).

We found that some of the tumors treated with retroviruses delivering *RB2*/*p130* underwent central necrosis and subsequent calcification.

### Tumor - Human Lung Adenocarcinoma and Glioblastoma

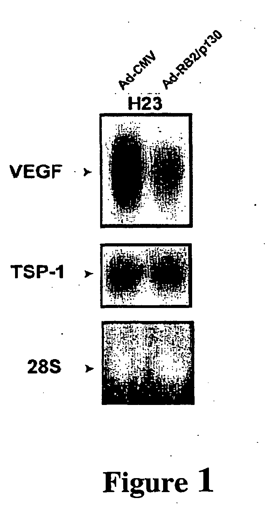

To show RB2/p130 expression down-regulates VEGF in tumor cells *in vitro* H23 cells were used. These cells were infected with recombinant viruses carrying *RB2*/*p130* or with the control recombinant virus and harvested following 48 hours. A down-regulation of 2 fold of the vascular endothelial growth factor upon over-expression of *RB2*/*p130* was achieved as shown by the northern blot analysis with a probe against VEGF (See, Fig. 1). Expression of *RB2*/*p130* brings about no modification of antiangiogenic proteins such as TSP-1. This was shown by the northern blot analysis with a probe against TSP-1 under the same experimental conditions (See, Fig. 1).

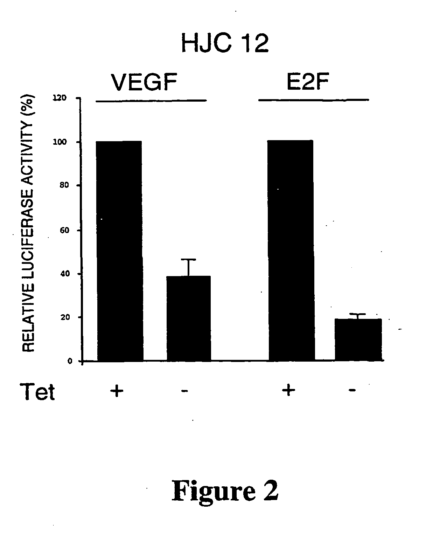

Low abundance of gene expression can result from either enhanced mRNA degradation or promoter regulation. The effects of forced *RB2*/*p130* gene expression on the VEGF promoter are also shown here. HJC #12 cells transfected with the VEGF promoter and cultured in the absence of the antibiotic tetracycline (RB2/p130 induced) showed 2-3 fold of down-regulation with respect to the un-induced HJC #12 (+ Tet) cells and to the vector control transfected cells in either the induced (- Tet) or un-induced status (+ Tet) (See, Fig 2). A promoter containing E2F consensus binding sites linked to the luciferase reporter gene was used as a positive control for pRb2/p130 transcriptional repression activity.

The VEGF protein abundance *in vitro* and *in vivo* following enhanced *RB2*/*p130* expression is also determined.

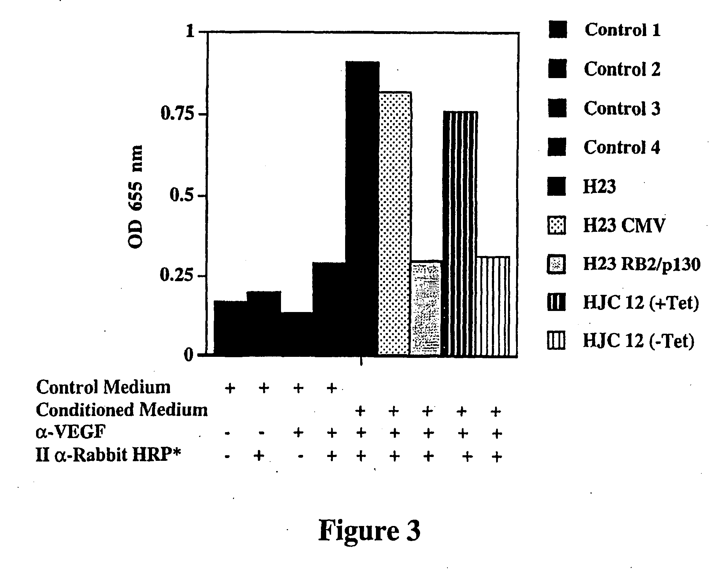

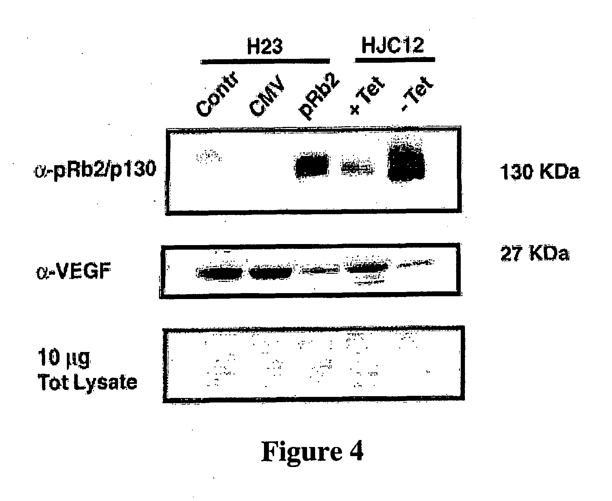

To show that RB2/p130 modulates VEGF protein expression *in vitro* the conditioned culture medium of H23 cells (Kondo et al., Biochim Biophys. Acta. 1221: 211-4, 1994) transiently transduced with either adenovirus carrying *RB2*/*p130* or with the CMV control adenovirus, were used and analyzed *i*.*e*., by means of an ELISA. Additionally, the conditioned medium of the HJC #12 cells in which the *RB2*/*p130* expression is regulated by a tetracycline inducible promoter was tested (Howard et al., J Natl Cancer Inst. 90: 1451-60, 1998). In both systems over-expression of *RB2*/*p130* resulted in a down-regulation of the VEGF protein abundance by 3 fold with respect to the controls (See, Fig. 3). VEGF abundance in the intracellular compartment was also tested. H23 cells were transiently transduced with either adenovirus carrying *RB2*/*p130* or with the CMV control adenovirus. The protein extracts from HJC #12 cells in which the *RB2*/*p130* expression is regulated by a tetracycline inducible promoter was also tested. Western Blot analysis using rabbit polyclonal antibodies against VEGF showed a 2-3 fold reduction of intracellular protein abundance upon enhanced RB2/p130 expression (See, Fig. 4).

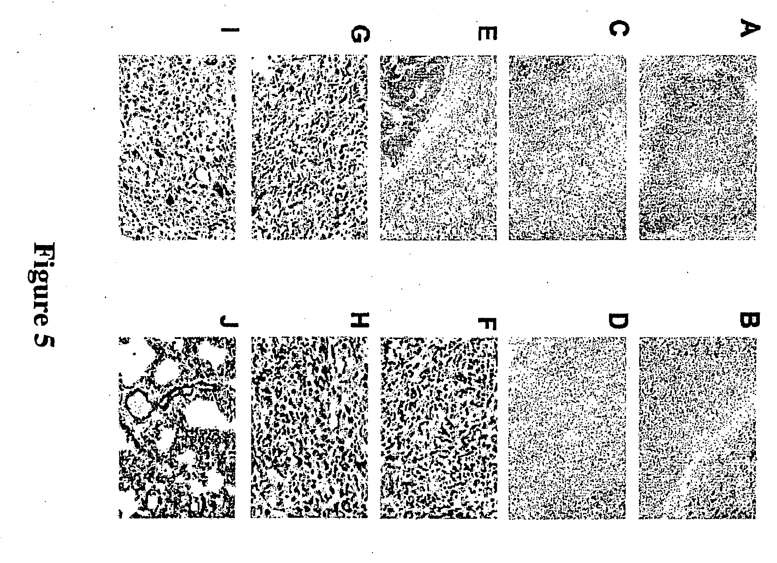

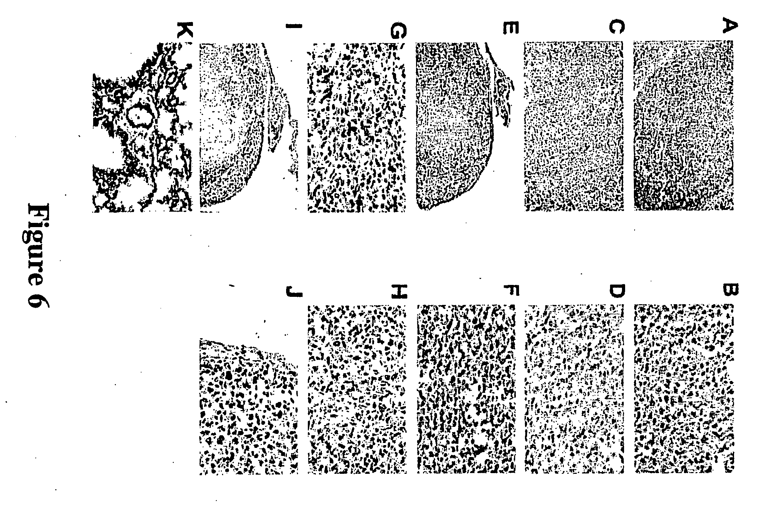

To show that RB2/p130 not only down-regulates VEGF expression but also inhibits angiogenesis *in vivo* tumors formed in nude mice were used as the target tissue area. The tumor formation in *nude* mice was carried out using the reported procedures. (Claudio, et al, Cancer Res. 60: 3 72-82, 2000; Howard et al., J Natl Cancer Inst. 90: 1451-60, 1998). Viral vectors expressing RB2/p130 were delivered into some of these tumors. Serial sections of tumors grown in *nude* mice and treated or untreated with *RB2*/*p130* were immuno-stained for VEGF and CD31. CD31 is a specific marker for endothelial cells (Horak et al., Lancet. 340: 1120-4, 1992) The VEGF staining was graded on a scale from 0 to 3 following previous work by Takahashi and colleagues, with some modification (Takahashi et al., Cancer Res. 55: 3964-8, 1995). The following grading was used a score of 0, equal to no detectable staining; 1, to traces of staining; 2, to a moderate amount of diffuse staining; and 3, to a large amount of diffuse staining. *RB2*/*p130* over-expression caused VEGF immunostaining to drop from a large amount of diffuse staining (score = 3), characteristic of the control samples, (See, Fig. 5 panels A, B, C and Fig. 6 panels A, B, C, D), to traces of staining (score = 0) (See, Fig.5 D and Fig. 6 panels E and F) in both the two tumor graft groups examined.

Intratumoral microvessel density assessment showed at least an 81% [CI = 1.95 - 10.5] reduction of microvessels count after CD31 immunostaining in all tumor grafts (H23 and HJC) in which *RB2*/*p130* was over-expressed. Referring now to Figures 5 and 6, in Fig. 5 panels F, G and H show a few representative examples of microvessel density in the HJC Δ5 (+Tet), HJC Δ5 (-Tet) and HJC #12 (+Tet) control tumor grafts, respectively. Fig. 6 panels G and H instead show samples of H23 tumor grafts treated with the control retroviruses carrying *Pac* or β-*Gal,* respectively. Fig. 5 I, however, demonstrates very poor microvessel density upon induction of *RB2*/*p130* expression in HJC #12 (-Tet) tumor grafts as evidenced by CD31 immunostaining. Fig 6 I at low magnification power (100X) shows poor microvessel density in a H23 tumor graft treated with retroviruses carrying *RB2*/*p130.* Additionally, Fig 6 I contains on its upper side a portion of normal *nude* mouse tissue demonstrating a normal neuro-vascular formation that was stained by the CD31 antibody, proving that the lack of CD31 staining is indeed specific to enhanced pRb2/p130 expression in tumor tissues. Fig. 6 J is a higher magnification field (400X) of panel I showing the only vascular formation present on this particular slide. Finally, figures 5J and 6K show the specificity of the CD31 staining to neuro-vascular bundles in normal embryonal mouse lung endothelium in the conditions used. The effects upon VEGF staining intensity and intratumoral microvessel density were specific to pRb2/p130 expression since withdrawal of tetracycline from the HJCΔ 5 tumors did not alter VEGF intensity and actually enhanced intratumoral microvessel density.

The analysis of results on VEGF staining and intratumoral microvessel density (IMD) (See Table 1 below) revealed that the induced or enhanced expression of RB2/p130 in tumor tissues resulted only in traces of VEGF staining. It correlated with very poor microvessel density in the respective samples.

**Table 1 VEGF staining intensity and (IMD) in nude mice tumors**

| **Tumor** | ***RB2*/*p130expression*** | **VEGF staining** **intensity** | **Microvessels/mm²** |
|---|---|---|---|
| HJC Δ5 (+ Tet) | Basal | 3 | 16 |
| HJC Δ5 (- Tet) | Basal | 3 | 34.16 |
| HJC 12 (+ Tet) | Basal | 2 | 19.2 |
| HJC 12 (- Tet) | Induced | 1 | 1.8 |
| H23 Pac | Basal | 3 | 10.6 |
| H23 β-Gal | Basal | 3 | 10.5 |
| H23 Rb2/p130 | Enhanced | 1 | 1.95 |

### Tumor - Glioblastoma

Malignant gliomas have extremely poor prognosis despite the use of currently available therapies such as surgery, radiation therapy, and chemotherapy. Gene therapy strategy based on the overexpression of Rb2/p130 can be used to interfere with the VEGF to block tumor angiogenesis and to inhibit tumor growth.

Recombinant vectors to deliver Rb2/p130 can be adeno or retroviral vectors as used for the preceding tumor tissue example. The recombinantretroviral vector can be used; the use of recombinant retroviral vector for gene therapy is known in the art. For example, the Rb₂/p130 can be subcloned into retroviral vector which is described further below in the Examples section. The following description of glioblastoma treatment is described using this recombinant vector as an example for Rb₂/p130 delivery.

Tumors can be established in a suitable animal model such as a rat. For example, GS9L (rat gliobastoma) cells can be used to establish tumors in rats. The GS9L cells are transplanted intracerebrally to establish intracerebral tumor model and subcutaneously to establish subcutaneous tumor model as described by Machein et al., Human Gene Therapy 10:1117-1128.

The recombinant virus vector can be delivered to the tumor cells in different forms known in the art. For example, the virus can be delivered by implanting virus producer cells (virus-packaging line) or by administering virus particle-containing producer cell supernatants to the established tumors.

It is preferred that the titer of virus-producing cell lines be in the range of 1 X 10¹⁰ to 1 X 10¹² CFU/ml. When virus-containing supernatant is used the preferred viral titer for retrovirus vector is 1 X 10⁶ to 1 X 10⁷ Pfu/ml viral vector or sufficient enough to transduce most of the endothelial cells in a growing tumor. The Rb2/p130 virus vector is administered to the tumor in a supernatant form or virus producer cell form during the early stage of tumor growth, preferably when the tumor is 25 mm³ after tumor cell inoculation. When virus producer cells are used to deliver Rb₂/p130, it is preferred to administer these cells in eight to ten-fold excess relative to the number of cells in tumor. The approximate cell number per tumor can be determined by measuring the size of the tumor and comparing it to the previously established tumor size and cell number correlation chart.

Animals are observed daily before and after the Rb₂/p130 treatments for the growth of tumors and symptoms associated with the progression of tumors. Expression of Rb₂/p130 and/or down-regulation of VEGF in tumors in the animals are determined by known techniques such as Southern blot analysis, Northern blot analysis, in situ hybridization and immunohistochemistry.

### Diabetic Retinopathy

pRb2/p130 is to be expressed in retinal tissue to down-regulate VEGF expression therein. It is known that VEGF causes retinal neovascularization in animals including human beings suffering from diabetic retinopathy. Diabetic retinopathy is a common microvascular complication in patients with type 1 diabetes. The progression of background retinopathy to proliferative retinopathy leads to visual impairment through bleeding or retinal detachment by accompanying fibrous tissues.

Experiment in animal models with induced ocular neovascularization are know to show that VEGF is upregulated severalfold before the formation of new blood vessels and that blocking its action inhibits retinal neovascularization.

Increased vascular permeability is a characteristic sign of early stages (background retinopanty) of diabetic retinopathy and VEGF is upregulated during this stage. Retinal digest preparations from diabetic animals and humans show scattered capillary occlusions which is a stimulus for increased vascular permeability. VEGF is a vascular permeability factor.

Diabetic rat model of experimental retinopathy is used for pRb2/p130 delivery and overexpression of this gene in the retinal tissue. Such diabetic rat model of retinopathy is known to one skilled in the art. For example, chronic hyperglycemia can be induced in 4-6 week old Wistar rats by intravenous injection of 60-65 mg/kg body weight streptozotocin. Diabetes can be monitored consecutively by taking body weight and blood glucose levels into consideration.

When these rats reach, for example, a body weight of about 330 g and their blood glucose levels of 25 nmol/1, pRb2/p130 can be administered to the retinal tissue at 1 to 2 week intervals. For each rat, a total of 1 x 10⁸ to 1 x 10¹⁰ pfu/ul of the recombinant adenoviral vector or a total of 1 X 10⁶ to 1 X 10⁷ pfu/ml retroviral vector expressing pRb2/-130 can be instilled into the retinal space. The age-matched nondiabetic rats are also used as controls. VEGF levels are monitored in the retinal tissues of diabetic and control rats at regular intervals of 7 to 14 days, by any of the suitable techniques such as in situ hybridization for VEGF, immunoreactivity, immunohistochemistry and western blot analysis. For example, retinal protein extracts can be performed to confirm the relative decrease in VEGF protein levels in retinal tissue. The treatments are continued until VEGF levels in the retinal extracts are similar to that in nondiabetic rats. Quantitation of cellular capillaries can also be performed in diabetic rats and compared to that of the controls. Thus pRb2/p130 gene therapy provides an effective anti-VEGF strategy in diabetic retinopathy

### Choroidal Neovascularization

In another aspect, the pharmaceutical composition, when administered can be used to inhibit choroidal neovascularization (CNV). CNV is a serious complication of age related macular degeneration and it is characterized by the growth of new blood vessels from the choroid, through the Buch's membrane into the subretinal space. This ultimately leads to the formation of choroidal neovascular membranes from which blood and serum may leak, causing vision loss. At present age-related macular degeneration is clinically difficult to treat

It is known that VEGF is a causative agent in a variety of ocular angiogenic diseases including age-related macular degeneration. For example, it has been shown that the overexpression of VEGF in retinal pigment epithelial cells is sufficient to induce CNV (Spilsbury et al. Am J Pathol 1257:135-144, 2000).

The animal models of choroidal neovascularization in the subretinal space are well known in the art (Tobe et al. J. Jpn Ophthalmol Soc 98:837-845, 1994; Shen et al., Br J Ophthamomol 82:1062-1071, 1998). For example, a rat with CNV can be administered with pRb2/p130 expressing vector. For example, a recombinant adenovirus vector can be used to specifically target the rat retinal pigment epithelium (RPE). Such vectors which specifically target RPE are known (Li et al., PNAS, 1995, 92:7700-7704; Rakoczy et al., Aust NX J Ophthalmol, 1998, 26:S56-S58). Such a recombinant adenovirus vector containing Rb2/p130 can be used to determine whether in vivo overexpression of Rb2/p130 in RPE cell is sufficient to down-regulate VEGF expression and inhibit CNV in the rat.

Briefly, the CNV rats can be used for subretinal injections of Ad-CMV-Rb2/p130 vectors. The animals are anesthetized, for example, by a mixture of ketamine and xylazine administered intramuscularly. The eyes can be further treated with topical amethocaine drops and the pupils dilated with 1% tropicamide and 2.5% phenylephrine hydrochloride drops. The conjunctiva can be cut close to the limbus to expose the sclera. A 32 gauge needle was then passed through this hole in a tangential direction under an operating microscope. 2 µl of Ad-CMV-Rb₂/p130, for example, at a dose of 4 X 10⁵, 4 X 10⁷, or 4 X 10⁹ pfu/eye is delivered to the subretinal space. Immediately after the subretinal injection a circular bleb is usually observed under the operating microscope. The success of each subretinal injection is further confirmed by the observation of a partial retina detachment as seen by indirect ophthalmoscopy. The needle is kept in the subretinal space for 1 minute, withdrawn gently, and antibiotic ointment applied to the wound site.

VEGF levels can be determined by VEGF mRNA expression in RPE cells and by histological analysis of Ad-CMV-Rb2/p130 injected in the eyes of these animals. In addition, to determine whether overexpression of Rb2/p130 in the RPE had down-regulated VEGF, which VEGF expression would otherwise have a vasopermeabilty effect on blood vessels, fluorescein angiograms can be used to detect vascular leakage. Fluorescein angiography in the context of CNV is well known in the art.

For example, fluorescein angiograms 5-10 days post-subretinal injection with the recombinant adenovirus can be performed to determine areas of vascular leakage.

Thus this Rb2/p130 provides an ideal system for targeted anti-angiogenic gene therapy in the eye.

### Rheumatiod Arthritis

In another aspect, the pharmaceutical composition, when administered can be used to down-regulate synovial fluid (SF) VEGF levels and prevention of pathological angiogenesis in Rheumatoid arthritis (RA) patients. Rheumatoid SF neutrophils have been shown to be the predominant source of VEGF in inflamed joints in RA (see Kasama et al., Clin Exp Immunol. 2000,121:533-538). In RA disease, synovial cells proliferate in response to inflammatory stimuli, which leads to the formation of a very aggressive invasive tissue, the rheumatoid pannus.

In the early states of synovitis, there is the development of new vessels in the synovium, which deliver nutrients, oxygen, and cells to the proliferating pannus. In mouse arthritis model, production of defectable levels of VEGF protein is associated with onset of clinical symptoms of arthritis. Similarly, in human RA disease, VEGF expression has been known to correlate with disease severity in patients with chronic RA (Paleolog *et al*. 1978) and, therefore, the prevention of the pannus induced bone erosions and loss of joint function. Inhibition of VEGF promoted angiogenesis in the synovium can provide a promising approach for the treatment of RA.

Animal models of RA are known in the art One such animal model is the KRN/NOD mouse model (Kouskoff et al., Cell, 1996, 87:811-822). The transgenic KRN/NOD mice develop arthritis. In these animals, the disease starts between 25 and 29 days after birth with a very acute stage characterized by joint effusions and florid synovitis that spread to all joints between days 27 and 36. The nontransgenic KRN/NOD mice remain in good condition with no signs of arthritis during this period.

The down-regulation of VEGF activity *in vivo* is achieved by administration of Rb2/p130. Rb2/p130 expressing vectors are delivered to synovial joints in order to transduce synovial cells, including leucocytes. The overexpression of Rb2/p130 in synovial cells down-regulates VEGF production. The vector can be delivered, for example, by direct injection into the synovium. For examples, the viral vector can be administered at a dose of 1 X 10⁸ to 1 X 10¹⁰ pfu/ml on the day of arthritis onset and every other day for 14 days at the same dose. Control animals receive the vector without Rb₂/P130 with the same dosing regimen. Throughout the disease duration the animals are scored for clinical symptoms of arthritis. In the control animals, arthritis development is unaltered. As part of the assessment, arthritis is quantified by measuring the thickness of each paw, for example, with a caliper-square. Then an arthritis index is calculated for each animal as the sum of the measures of the paws.

Some of the joints into which vector can be delivered for treatment and analyzed after the treatment are wrist, ankle, knee, shoulder, elbow, metacarpophalangeal, metatarsophalangeal and hip joints. Improvement in histologic features of arthritis after administration of Rb₂/p130 is analyzed. Tendon ruptures, synovial membranes invaded by the inflammatory materials, articular space filled with inflammatory materials, severe destructive lesions of the tarsal and carpal joints, panus proliferation and invasion, very intense bone lesions in terms of bone or cartilage destruction, fibrosis and fusion are some of the features of arthritis which are seen in the control RA animals but should be absent or should be seen with reduced severity in Rb₂/130 treated animals. In other words, administration of Rb₂/130 reduces the clinical score as well as the extent of synovitis and joint destruction, which is indicative of a suppression of the formation of the pannus. Since blood vessels are required to nourish and maintain the pannus, inhibiting angiogenesis and snyovial mass is almost certainly associated with a decrease in the total number of blood vessels.

The acute-phase response, as measured by C-reactive protein (CRP), is a marker for RA disease activity and is commonly used in clinical practice to monitor RA disease activity. Elevated levels of CRP are generally indicative of disease progression and lack of improvement under therapy. It is known that serum CRP levels significantly correlate with both cell-associated and free VEGF in SF, thereby providing a useful method for monitoring the disease activity of RA. Also, serum VEGF concentration has been reposted to correlate with serum CRP level and RA activity correlates with serum concentration of CRP (Harada et al., Scand J Rheumatol, 1998 27:377-380), and therefore serum VEGF level represents the disease activity of RA.

Thus, the present invention provides the use of a vector expressing Rb2/p130 gene for down-regulating angiogenetic factors to inhibit angiogenesis *in vivo* . The vectors can be a viral or a non-viral vector (e.g. a naked plasmid). In a preferred embodiment the Rb2/p130 full length gene is subcloned into a viral vector. By following the methods of vector construction known to make a recombinant vector selection of an appropriate vector can be based upon an adeareate expression of the gene in the transferred cells. Among viral vectors, adenoviral and retroviral vectors are preferred. One skilled in the art would also know how to use nonviral vectors such as eukaryotic expression plasmids to express Rb2/p130 in desired tissues in quantities sufficient to achieve the down-regulation of angiogenic factor to inhibit angiogenesis.

The selected recombinant vector is then transfected or delivered into the target tissue area by using methods known to those of skill in the art. The transfection and or delivery methods may vary depending on the target tissue area requiring the inhibition of pathological angiogenesis direct injection of the recombinant vector(s) into the target area may be employed as a delivery method. For example, in the case of tumors, the patient can receive injections of the vector directly into the tumor tissue. Other methods such as encapsulation in various forms of liposomes, tissue specific delivery and particle bombardment may also be used depending on the location of the target tissue area in a mammalian body such as human.

Tissue-specific delivery of Rb₂/130 is achieved by a number of ways. One approach is to use tissue-specific promoter to control transgene expression. Another approach is to manipulate the vector particle itself so that it selectively targets and transduces only certain cell types. Still another approach is the use of microbubble directed gene delivery to a specific tissue.

There are a number of tissue specific promoters known in the art. Bone tissue specific promoters (Stein et al., Cancer, 2000, 88:2899-2902) for the treatment of tumor metastasis to the bone. Prostate specific antigen (PSA) promoter for the gene therapy of prostate cancer (Lee et al., Anticancer Research, 2000, 20:417-422) promoters active only in proliferating cells (Nettelbeck et al., 1999, Gene Therapy 6:1276-1281); hepatocarcinoma-specific alpha-fetoprotein (AFP) promoter (Sato et al., Biochem. Biophys. Res. Comm., 1998, 244:455-462).

Ultrasound-mediated microbubble destruction can be an effective method for the tissue-specific delivery of Rb₂/130. This method is known to one skilled in the art. For example, there has been successful demonstrations in the prior art that the ultrasound-mediated disruption of gas-filled microbubbles can be used to direct transgene expression to a specific tissue (See Shohet et al., Circulation, 2000, 101(22): 2554-2556).

It is preferred that the vector used in the present invention is to be administered in a pharmaceutically acceptable carrier. The carriers suitable for administration include (pharmacologically or physiologically acceptable) aqueous and non-aqueous sterile injection solutions which may contain buffers, antibiotics and solutes which render the carrier isotonic with the bodily fluid of the patient. The carrier formulation, dose of the vector and duration of treatment is determined individually depending on the need to inhibit angiogenesis. Such determination can be made by those skilled in the art.

### EXAMPLES

The examples below are carried out using standard techniques, that are well known and routine to those of skill in the art, except where otherwise described in detail. The examples illustrate the invention. All animal methods of treatment or prevention described herein are preferably applied to mammals, most preferably to humans.

### Example 1: Construction of the RB2/p130 and control vectors

Retroviral and adenoviral vectors expressing *RB2*/*p130* or controls expressing the bacterial β-galactosidase (*Lac-Z*) or the puromycin resistance (*Pac*) gene alone have been previously described in the art (Claudio et al., Cancer Res. 60: 372-82, 2000; Claudio et al., Circ Res. 85: 1032-9,1999). The retroviral-mediated gene transfer studies were carried out with a murine leukemia virus (MLV)-based system (Claudio et al., Cancer Res. 60: 372-82, 2000). A transient three-plasmid expression system was used for the production of high titer retroviral vectors. This system consisted of MLV-based retroviral vectors with their packaging components expressed from the strong CMV promoter and carried on plasmids containing SV40 origins of replication, which enhances retroviral gene expression in cell lines carrying the SV40 large T antigen. To reduce the risk of helper virus formation the two packaging components, *gag-pol* and *env*, were cloned into separate plasmids. The *env* expression plasmid is devoid of its 3'LTR sequences that were homologous to a region of the *gag-pol* expression plasmid to prevent helper virus formation through recombination events. The *env* and *gag-pol* plasmids encode aa RNA transcript which was substrate only for translation within 293T cells which are highly transfectable and contain the large T antigen. A third plasmid produced a chimeric "proviral" RNA genome, which was substrate for packaging into the virion, for reverse transcription and for integration into the host genome. This plasmid contained the CMV promoter, driving the 5'MLV-LTR, a cassette (anything), the SV40 promoter, the neomycin resistance gene, and the 3'MLV-LTR; and the backbone of this plasmid has the SV40 ori. Sodium butyrate (NaB) was added to the medium for 12-14 hours at a final concentration of 10 mM after the removal of the calcium phosphate-DNA co-precipitate to increase viral titers by almost a log. Fresh medium was then added and the supernatants were harvested 12 hours later. NaB increased the percentage of cells expressing exogenous DNA, and NaB activated several eukaryotic promoters, including the CMV promoter.

The following plasmids were generated: pHIT456--CMV-MLV amphotropic env-SV40ori; pHIT123--CMV-MLV ecotropic env-SV40ori; pHIT60--CMV-MLV gag-pol-SV40ori; pHIT111--CMV-LacZ-SV40 promoter-NEO-SV40ori; MCSV-Pac; MCSV-neo.

The MSCV plasmids were taken and inserted the full length cDNA of the RB2/p130 gene to form the MSCV-pRb2/p130 construct. The *neo or Pac* gene allowed selection for successfully transduced cells by addition of G418 or puromycin to their medium. The pHIT111 plasmid (Retro-β-gal) containing the bacterial β-galactosidase (LacZ) gene was used as a control to assay the effects of the viral vector alone, independent of RB2/p130, on tumor suppression. The ecotropic envelope, plasmid pHIT123, was used for safety concerns to produce the virus for the studies in rodent tumors and rodent cell lines. The amphotropic envelope, plasmid pHIT456, was used to produce the virus for studies in human tumor cell lines. The transient three-plasmid expression system was used for the production of high titer retroviral vectors and in the transduction of tumor cell lines in culture as well as of *in vivo* tumors. Transient and DNA cotransfection of the 293T/17 cells using PHIT60 (CMV-MLV-gag-pol-SV40 ori) and PHIT456 (CMV-MLV-amphotropic env-SV40 ori) vectors along with murine stem cell virus (MSCV)-based transfer vectors MSCV-*Pac*, MSCV-*Pac-LacZ, MSCV-Pac-RB2*/*p130* were performed by calcium phosphate precipitation (Claudio et al., Cancer Res. 60: 372-82, 2000).

The retroviral supernatant was collected 48 hours post-transfection, filtered through 0.45 µm filters and titered as previously described (Claudio et al., Cancer Res. 60: 372-82, 2000) to produce retroviruses carrying the puromycin resistance gene alone or in combination with the *Lac-Z* gene or the *RB2*/*p130* open reading frame (ORF), respectively. Viral titers of 1 x 10⁷ infectious units/mL were obtained (Claudio et al., Cancer Res. 60: 372-82, 2000).

Adenoviruses were generated by sub-cloning the full length ORF of the *RB2*/*p130* gene into the pAd.CMV-Link1 vector to form the Ad.CMV*-RB2*/*p130* virus as previously described (Claudio et al., Circ Res. 85: 1032-9, 1999). The pAd.CMV-Link1 vector alone (to produce the Ad-CMV virus) was used as a negative control to assay the effects of viral infection alone without delivering a transgene. The above mentioned viruses were generated by co-transfection of the previously mentioned constructs with an adenoviral backbone into the packaging cell line 293 primary embryonal human kidney cells, transformed by sheared human adenovirus type 5. The adenoviruses were recovered, screened and expanded as previously described (Claudio et al., Circ Res. 85: 1032-9, 1999). Following purification by sequential equilibrium density gradients using CsCl viral stocks were made at 5 x 10¹² particles/mL and stored at -80 C in a solution containing 10% glycerol. A viral titer of 22 x 10⁹ pfu/mL was determined by plaque assay for the Ad-CMV and Ad-CMV*-RB2*/*p130* viruses. Infection of nonpermissive cells confirmed that the viruses were replication defective.

### Example 2: Effect of RB2/p130 on VEGF expression in vitro

H23 cells (Human Lung Adenocarcinoma) have been previously described (28). HJCΔ5 cells and its clone HJC#12 (JC-Tantigen transformed hamster glioblastoma) expressing pRb2/p130 under an inducible tetracycline promoter have been previously described. Howard et al., J Natl Cancer Inst. 90: 1451-60, 1998. Briefly, we utilized a modified tetracycline-regulated method to create an autoregulatory inducible *RB2*/*p130* gene expression system created in the HJC-15c cell line, originating from a human polyomavirus-induced (JC virus) hamster brain tumor. Howard et al., J Natl Cancer Inst. 90: 1451-60, 1998. The parental cell line HJC-15c was used to create the control cell line HJCΔ5 that contains the tetracycline transactivator (tTA) under the control of the Tetp promoter. HJCΔ5 cells were used to form the HJC #12 cell line, which contains, in addition to tTA, the full length cDNA of the human *RB2*/*p130* gene downstream of the tetp promoter. In this system Rb2/p130 expression is repressed in the presence of the antibiotic tetracycline (+) and induced in its absence (-) to 100 fold at the protein level (29). The 293T/17 cell line (human renal carcinoma), (Stiegler et al., J Cell Biochem Suppl. 31: 30-6, 1998), was purchased from the American Type Culture Collection (ATCC) upon authorization of the Rockefeller University. H23 cells were maintained in Dulbecco's modified Eagle medium (D-MEM) supplemented with 10% fetal bovine serum (FBS), 2 mM 1-glutamine. The 293T/17 cell line was maintained in DMEM supplemented with 10% heat inactivated FBS and 2 mM 1-glutamine. HJCΔ5 and HJC#12 cells were grown in Dulbecco's Modified Eagle's Media (DMEM) supplemented with 5% fetal calf serum (Sigma St. Louis, MO) and the antibiotics streptomycin (10mg/mL) and penicillin (100 units/mL) and in the presence or not (+/-) of 2 µg/mL of tetracycline (Sigma, St. Louis, MO).

### Example 3: Effect of RB2/p130 on VEGF expression and angiogenesis

Tumors were generated by the subcutaneous injection of 2.5 X 10⁶ H23 or of 5 X 10⁶ HCJ Δ5 or HJC#12 cells, into *nude* mice (female NU/NU-*nu*BR outbred, isolator-maintained mice, 4-5 weeks old from Charles Rivers Wilmington, MA), as previously described (Claudio et al., Cancer Research:60-372-382, 2000, Howard et al., J. Nat'l Cancer Inst., 90:1451-60, 1998).

For H23 injected cells, when the tumors reached a volume of approximately 20 mm³ after 15 days, each tumor was transduced with 5 X 10⁶ retroviruses carrying the *Pac* gene alone or the *Pac* gene and the *Escherichia coli* β-galactosidase (*LacZ*) gene as control or the *Pac* gene and *RB2*/*p130* open reading frame (ORF) with three animals per group by direct injection of 20 µL of retroviral supernatant directly into each of the tumors.

For the HJC *nude* mice group the mice were treated with tetracycline for 4 days prior to injection. The mice were injected subcutaneously along their left and right flanks at two sites per mouse with 5 X 10⁶ cells per flank while under anesthesia with isopropane gas. There were four groups of animals with three animals per group. Two groups were injected with HJC12 cells and treatment with tetracycline continued following injection in one group (12+), whereas another group (12-) ceased to be administered tetracycline after injection of the cells. The two control groups were injected with HJCΔ5 cells and one (Δ5+) continued to receive tetracycline while the other control group (Δ5-) did not.

Animals were sacrificed by CO₂ asphyxiation when *Pac* and *LacZ* retroviral-transduced tumors or HJC Δ5 (± tetracycline) and HJC #12 tumors (+ tetracycline) reached a size of 300-350 mm³. Tissues to be sectioned were placed in OTC (Sakura Finetek USA, Inc., Torrance, CA), frozen in liquid nitrogen, and stored at -80 °C or preserved in neutral-buffered formalin at 4 °C before embedding in paraffin.

### Example 4: Analysis of the effect of RB21/p130 on VEGF expression and angiogenesis

### a) Northern blot analysis

Shown in Figure 1 is Northern blot analysis of H23 cells transduced with either Ad-CMV or Ad-*RB2*/*p130*. The probes used are indicated on the left. The lower panel shows the ethidium bromide staining for equal gel loading. Two fold reduction of VEGF abundance was observed upon enhanced *RB2*/*p130*expression.H23 cells were grown to 70% confluency then infected with 50 MOI of adenoviruses carrying *RB2*/*p130* ORF or with the control adeno-CMV. After 14 hours the medium was changed, and the cells were harvested after a total of 48 hours post infection.

VEGF northern blot analysis was performed essentially as previously described. Rak et al., Cancer Res. 55: 4575-80, 1995. Briefly, RNA was extracted using the RNAzol kit (TEL-TEST, Inc., Friendswood, TX) following the manufacturer's instructions. The RNA was resolved on a 1% agarose gel containing 6.6 M formaldehyde, transferred to a Zeta Probe (Bio-Rad, Hercules, CA) membrane, and hybridized at 65 °C with a ³²P-labeled cDNA probe containing either the 200 bp fragment of the human VEGF sequence common to all four known isoforms of VPF/VEGF protein or TSP-1. Rak et al., Cancer Res. 55: 4575-80, 1995; Berse et al., Mol. Biol. Cell. 3: 211-220, 1992. The amount of RNA loaded in each lane was evaluated by ethidium bromide gel staining of the gel before the transfer. The TSP-1 probe was purchased from the ATCC.

### b) Luciferase Assay

Shown in Figure 2 is a bar graph illustrating VEGF luciferase activity in HJC#12 cells VEGF and E2F promoter luciferase constructs were transiently transfected into HJC#12 cells and subsequently pRb2/p130 expression was induced (-Tet). The promoters used are indicated on the top. A 2-3 fold reduction of VEGF promoter activity was observed upon enhanced *RB2*/*p130*expression.

Luciferase assay was performed by transfecting a total of 3 µg of either mouse VEGF promoter (Su et al., Proc. Natl. Acad. Science USA. 96: 15115-20, 1999) or an artificial E2F promoter containing 3 consecutive E2F consensus binding sites (Magnaghi-Jaulin et al., Nature 391: 601-4, 1998) linked to the luciferase reporter gene for each point in the HJC # 12 cells, in the presence of the antibiotic tetracycline (+) (un-induced status) and in its absence (-) (induced pRb2/p130 protein status). HJC #12 cells were plated at 60% confluency in six-well dishes the day before the experiment and transfections were performed by the standard calcium-phosphate method as previously reported. Howard et al., J Natl Cancer Inst. 90: 1451-60, 1998. Normalization was performed by co-transfecting a total of 1 µg of CMV *Lac-Z* (Promega, CA) for each experimental point. The experiment was performed in triplicates and repeated twice. Luciferase activity was assayed using the luciferase kit assay according to the manufacturer's instructions (Promega, Madison, WI) and measured using a luminometer (Corning Costar Corp., Cambridge, MA).

### c) Enzyme-Linked-Immunosorbent-Assay (ELISA)

The VEGF ELISA was performed as previously described using the anti-VEGF from Genentech, Inc. (San Francisco, CA) (1:2000 dilution) and an anti-rabbit HRP (horse radish peroxidase) conjugated antibody (Hamersham, Arlington Heights, IL) (1:5000 diluton) as secondary antibody and the *3,3',5'5-tetramethylbenzidine* (TMB) liquid substrate system following the manufacturer's recommendations (Sigma, Saint Louis, MO). Dirix et al., Br J Cancer. 76: 238-43, 1997. A Graphic representation of a single experiment of a VEGF Enzyme-Linked-Immunosorbent-Assay (ELISA) in the conditioned medium of H23 and HJC#12 cells following *RB2*/*p130* overexpression is shown in Figure 3. Lanes 1 to 4 control medium; lanes 5 to 9 conditioned medium. Lanes 1 to 3 negative controls. Lane 4 represents the background. Bars are labeled on the right. The graph is the representation of a single experiment that was repeated 3 times with the same result.

### d) Western Blot

Protein concentration was assayed by Bradford analysis (Bio-Rad Laboratories, Inc., Melvile, New York) and confirmed by running 10 µg of protein on a 12% sodium dodecyl sulfate-polyacrylamide gel (SDS-PAGE), and staining with Coomassie blue. For Western blotting purposes an equal amount of 100 µg of protein extract for each sample was electrophoresed into 12% sodium dodecyl sulfate-polyacrylamide gels (SDS-PAGE) and transferred to 0.2 µm nitrocellulose membranes (Schleicher & Schuell, Germany). The loading and transfer of equal amounts of protein was confirmed by staining the membranes with Red Ponceau (Sigma, St. Louis, MO). Membranes were quenched at 4 °C overnight in a solution of TBS-T (Tris-buffered Saline + 0.5% Tween-20) and 5% dry milk for blocking nonspecific binding. Either primary rabbit polyclonal anti-VEGF (Genentech,San Francisco, CA) or anti-VEGF (Santa Cruz Biotechnology Inc., Santa Cruz, CA) diluted 1:200 in a solution of TBS-T and 3% dry milk was used independently to incubate the blots. After several washes in a solution of TBS-T the blots were incubated with a solution of TBS-T containing a anti-rabbit secondary antibody horseradish peroxidase conjugated (Amersham, Life Science) diluted 1:20,000 for 1 hour at room temperature. The blots were then washed several times in TBS-T, reacted with a ECL (Chemoluminescence Kit from NEN, Boston, MA), and exposed to x-ray films.

Western blot analysis of VEGF protein abundance upon pRb2/p130 enhanced expression in H23 and HJC#12 cells Is shown in Figure 4. Cell lines are indicated on the top. H23 cells were transduced with either adenoviral vector carrying RB2/p130 (pRb2) or empty adenoviral vector (CMV). HJC#12 cells were grown under an induced (-Tet) or uninduced (+Tet) condition. A 3 fold reduction of VEGF protein abundance was observed upon enhanced *RB2*/*p130*expression. Comassie blue staining of 10 µg protein of total lysate is shown to verify protein concentration and equal loading.

### e) Antibodies, immunohistochemical analysis and intratumoral microvessel density assessment (IMD)

Rabbit polyclonal anti VEGF was obtained from Genentech, Inc. (San Francisco, CA). Purified anti-mouse CD31 (PECAM-1), clone MEC 13.3 was purchased from (Pharmingen, San Diego, CA). Anti VEGF was used at a dilution 1:500 and anti CD31 at a dilution of 1:50 following the manufacturer's instructions for immunohistochemical analysis.

VEGF staining intensity was graded on a scale of 0 to 3: 0, being no detectable staining; 1, traces of staining; 2, moderate amount of diffuse staining; and 3, a large amount of diffuse staining. This grading scale is a modification of the prior art known method (Takahashi et al., Cancer Res. 55: 3964-8, 1995).

Intratumoral microvessels were highlighted by immunostaining different serial formalin-fixed, paraffin-embedded sections of the same tumor graft with anti-CD31. IMD (intratumoral microvessel density) was determined as previously described. Vermeulen et al., Eur J Cancer. 32A: 2474-84, 1996. Briefly, CD31 stained sections underwent an individual microvessel count on a 400 X magnification in the areas of most intense neo-vascularization (hot spots). IMD was expressed as microvessels/mm².

Shown in Figure 5 is immunohistochemical analysis of VEGF and CD31 of HJC Δ5 and HJC#12 tumor grafts grown in *nude* mice **A)** High VEGF expression in HJC Δ5 (+ Tet) tumor [control] (100X); **B)** High VEGF expression in HJC Δ5 (- Tet) tumor [control] (100X); **C)** High VEGF expression in HJC #12 (+ Tet, pRb2/p130 not induced) tumor [control] (100X); **D)** Low VEGF expression in HJC#12 (- Tet, pRb2/p130 induced) tumor (100X); **E)** VEGF expression in a human colon cancer: the lower left corner shows high VEGF expression in the tumor, while the upper right corner shows low VEGF expression in the normal colon tissue (100X). **F)** CD31 immunostaining of HJC Δ5 (+ Tet) tumor [control]; **G)** CD31 immunostaining of HJC Δ5 (- Tet) tumor [control] (400X); **H)** CD31 immunostaining of HJC #12 (+ Tet, pRb2/p130 not induced) tumor [control] (400X); **I**) CD31 immunostaining of HJC #12 (- Tet, pRb2/p130 induced) tumor (400X); **J)** CD31 immunostaining of normal mouse lung (400X).

Shown in Figure 6 is Immunohistochemical analysis of VEGF and CD31 of H23 tumor grafts grown in *nude* mice **A**) High VEGF expression in H23 tumor transduced with control retrovirus (*Pac*) (100X); **B)** High power field (400X) of panel **A C**) High VEGF expression in H23 tumor transduced with retrovirus carrying *LacZ* (100X); **D** High power field (400X) of panel B; **E)** Low VEGF expression in H23 tumors transduced with retrovirus carrying *RB2*/*p130*. Upper side of the panel shows a normal mouse neuro-vascular formation. (100X); **F)** High power field (400X) of panel E showing the lack of VEGF immunostaining; **G)** CD31 immunostaining of H23 tumor transduced with control retrovirus (*Pac*) (400X); **H)** CD31 immunostaining of H23 tumor transduced with retrovirus carrying *LacZ* (400X); **I)** CD31 immunostaining of H23 tumors transduced with retrovirus carrying *RB2*/*p130* (100X); Upper side of the panel shows a normal mouse neuro-vascular formation stained for CD31. **J)** High power field (400X) of panel I showing the only vessels found in the slide. **K)** CD31 immunostaining of normal mouse lung (400X).

## Claims

1. Use of a vector expressing pRb2/p130 for the preparation of a pharmaceutical composition for the inhibition of angiogenesis in a target tissue of a patient in need of said inhibition.

2. A vector expressing pRb2/p130 for use in inhibiting angiogenesis in a target tissue.

3. The use of claim 1 or the vector of claim 2, wherein the target tissue is a retinal tissue, a retinal pigment epithelium, a synovial tissue, a tumor or cancer.

4. The use of claim 1 or 3 or the vector of claim 2 or 3, wherein the vector is a retroviral vector.

5. The use of claim 1 or 3 or the vector of claim 2 or 3, wherein the vector is an adenoviral vector.

6. The use of any one of claims 1 and 3 to 5 or the vector of any one of claims 2 to 5, wherein said expression of pRb2/p130 in the target tissue causes the down-regulation of VEGF in the target area.

7. Use of a recombinant viral vector expressing Rb2/p130 or a fragment thereof for the preparation of a pharmaceutical composition for the specific modulation of the expression of a gene of interest in one or more cells, wherein the gene encodes a protein, the expression of which is associated with angiogenesis within a patient.

8. A recombinant viral vector expressing Rb2/p130 or a fragment thereof for use in specifically modulating the expression of a gene of interest in one or more cells, wherein the gene encodes a protein, the expression of which is associated with angiogenesis.

9. The use of claim 7 or the recombinant viral vector of claim 8, wherein the gene of interest is VEGF.

10. The use or the recombinant viral vector of claim 9, wherein Rb2/p130 or a fragment thereof interferes with promoter regulation of said VEGF.

11. The use or the recombinant viral vector of claim 9, wherein Rb2/p130 or a fragment thereof interferes with mRNA expression of said VEGF.

12. The use or the recombinant viral vector of claim 9, wherein Rb2/p130 or a fragment thereof interferes with protein expression of said VEGF.

13. The use of any one of claims 7 and 9 to 12 or the recombinant viral vector of any one of claims 8 to 12, wherein said cells are cells of a human tumor.

14. The use of any one of claims 7 and 9 to 12 or the recombinant viral vector of any one of claims 8 to 12, wherein said cells are cells of a human cancer.

15. The use of any one of claims 7 and 9 to 12 or the recombinant viral vector of any one of claims 8 to 12, wherein said cells are cells of a retina, retinal pigment epithelium, synovial leucocytes or synovial neutrophils.

16. Use of a recombinant vector expressing pRb2/p130 for the preparation of a pharmaceutical composition for the inhibition of angiogenesis in a cancer tissue of a patient to treat cancer, wherein the vector is an adenoviral vector or a retroviral vector.

17. A recombinant vector expressing pRb2/p130 for use in inhibiting angiogenesis in a cancer tissue of a patient to treat cancer, wherein the vector is an adenoviral vector or a retroviral vector.

18. The use or the vector of any one of claims 3 to 6, 14, 16 or 17, wherein the cancer is human glioblastoma, melanoma, breast cancer, prostate cancer, lung cancer, endometrial cancer or stomach carcinoma.

19. Use of a recombinant vector expressing pRb2/p130 for the preparation of a pharmaceutical composition for the inhibition of angiogenesis in lung cancer tissue of a patient, wherein the dosage of said composition is sufficient to down-regulate VEGF expression so as to inhibit angiogenesis in said tissue, and the vector is an adenoviral vector or a retroviral vector.

20. A recombinant vector expressing pRb2/p130 for use in inhibiting angiogenesis in lung cancer tissue of a patient, wherein the dosage of said composition is sufficient to down-regulate VEGF expression so as to inhibit angiogenesis in said tissue, and the vector is an adenoviral vector or a retroviral vector.

21. Use of a recombinant vector expressing pRb2/p130 for the preparation of a pharmaceutical composition for the inhibition of angiogenesis in glioblastoma tissue of a patient, wherein the dosage of said composition is sufficient to down-regulate VEGF expression so as to inhibit angiogenesis in said tissue and the vector is an adenoviral vector or a retroviral vector.

22. A recombinant vector expressing pRb2/p130 for use in inhibiting angiogenesis in glioblastoma tissue of a patient, wherein the dosage of said composition is sufficient to down-regulate VEGF expression so as to inhibit angiogenesis in said tissue and the vector is an adenoviral vector or a retroviral vector.

23. Use of a recombinant vector expressing pRb2/p130 for the preparation of a pharmaceutical composition for the inhibition of angiogenesis in prostate cancer tissue of a patient, wherein the dosage of said composition is sufficient to down-regulate VEGF expression so as to inhibit angiogenesis in said tissue, and the vector is an adenoviral vector or a retroviral vector.

24. A recombinant vector expressing pRb2/p130 for use in inhibiting angiogenesis in prostate cancer tissue of a patient, wherein the dosage of said composition is sufficient to down-regulate VEGF expression so as to inhibit angiogenesis in said tissue, and the vector is an adenoviral vector or a retroviral vector.

25. Use of a recombinant vector expressing pRb2/p130 for the preparation of a pharmaceutical composition for the treatment of rheumatoid arthritis in a patient, wherein the dosage of said composition is sufficient to down-regulate VEGF expression in synovial tissue and inhibit angiogenesis in said synovial tissue, and the vector is an adenoviral vector or a retroviral vector.

26. A recombinant vector expressing pRb2/p130 for use in treating rheumatoid arthritis in a patient, wherein the dosage of said composition is sufficient to down-regulate VEGF expression in synovial tissue and inhibit angiogenesis in said synovial tissue, and the vector is an adenoviral vector or a retroviral vector.

27. Use of a recombinant vector expressing pRb2/p130 for the preparation of a pharmaceutical composition for the treatment of diabetic retinopathy in a patient, wherein the dosage of said composition is sufficient to down-regulate VEGF expression in the retina and inhibit angiogenesis in the retina, and the vector is an adenoviral vector or a retroviral vector.

28. A recombinant vector expressing pRb2/p130 for use in treating diabetic retinopathy in a patient, wherein the dosage of said composition is sufficient to down-regulate VEGF expression in the retina and inhibit angiogenesis in the retina, and the vector is an adenoviral vector or a retroviral vector.

29. Use of a recombinant vector expressing pRb2/p130 for the preparation of a pharmaceutical composition for the treatment of choroidal neovascularization in a patient, wherein the dosage of said composition is sufficient to down-regulate VEGF expression in said tissue and inhibit angiogenesis in the retina, and the vector is an adenoviral vector or a retroviral vector.

30. A recombinant vector expressing pRb2/p130 for use in treating choroidal neovascularization in a patient, wherein the dosage of said composition is sufficient to down-regulate VEGF expression in said tissue and inhibit angiogenesis in the retina, and the vector is an adenoviral vector or a retroviral vector.

## Patentansprüche

1. Verwendung eines Vektors, der pRb2/p130 exprimiert, zur Herstellung einer pharmazeutischen Zusammensetzung zur Inhibierung der Angiogenese in einem Zielgewebe eines Patienten, der diese Inhibierung benötigt.

2. Vektor, der pRb2/p130 exprimiert, zur Verwendung in der Inhibierung der Angiogenese in einem Zielgewebe.

3. Verwendung nach Anspruch 1 oder Vektor nach Anspruch 2, wobei das Zielgewebe ein Netzhautgewebe, ein Netzhautpigmentepithel, ein synoviales Gewebe, ein Tumor oder Krebs ist.

4. Verwendung nach Anspruch 1 oder 3 oder Vektor nach Anspruch 2 oder 3, wobei der Vektor ein retroviraler Vektor ist.

5. Verwendung nach Anspruch 1 oder 3 oder Vektor nach Anspruch 2 oder 3, wobei der Vektor ein adenoviraler Vektor ist.

6. Verwendung nach einem der Ansprüche 1 und 3 bis 5 oder Vektor nach einem der Ansprüche 2 bis 5, wobei die Expression von pRb2/p130 in dem Zielgewebe die Herunterregulierung von VEGF in dem Zielbereich bewirkt.

7. Verwendung eines rekombinanten viralen Vektors, der pRb2/p130 exprimiert, oder eines Fragments davon zur Herstellung einer pharmazeutischen Zusammensetzung zur spezifischen Modulierung der Expression eines Gens von Interesse in einer oder mehreren Zellen, wobei das Gen für ein Protein kodiert, dessen Expression mit der Angiogenese in einem Patienten assoziiert ist.

8. Rekombinanter viraler Vektor, der pRb2/p130 exprimiert, oder ein Fragment davon zur Verwendung in der spezifischen Modulierung der Expression eines Gens von Interesse in einer oder mehreren Zellen, wobei das Gen für ein Protein kodiert, dessen Expression mit Angiogenese assoziiert ist.

9. Verwendung nach Anspruch 7 oder rekombinanter viraler Vektor nach Anspruch 8, wobei das Gen von Interesse VEGF ist.

10. Verwendung oder rekombinanter viraler Vektor nach Anspruch 9, wobei pRb2/p130 oder ein Fragment davon die Regulierung des Promotors von VEGF behindert.

11. Verwendung oder rekombinanter viraler Vektor nach Anspruch 9, wobei pRb2/p130 oder ein Fragment davon die mRNA-Expression von VEGF behindert.

12. Verwendung oder rekombinanter viraler Vektor nach Anspruch 9, wobei pRb2/p130 oder ein Fragment davon die Proteinexpression von VEGF behindert.

13. Verwendung nach einem der Ansprüche 7 und 9 bis 12 oder rekombinanter viraler Vektor nach einem der Ansprüche 8 bis 12, wobei die Zellen Zellen eines menschlichen Tumors sind.

14. Verwendung nach einem der Ansprüche 7 und 9 bis 12 oder rekombinanter viraler Vektor nach einem der Ansprüche 8 bis 12, wobei die Zellen menschliche Krebszellen sind.

15. Verwendung nach einem der Ansprüche 7 und 9 bis 12 oder rekombinanter viraler Vektor nach einem der Ansprüche 8 bis 12, wobei die Zellen Zellen einer Netzhaut, von Netzhautpigmentepithel, synoviale Leukozyten oder synoviale Neutrophile sind.

16. Verwendung eines rekombinanten Vektors, der pRb2/p130 exprimiert, zur Herstellung einer pharmazeutischen Zusammensetzung zur Inhibierung der Angiogenese in einem Krebsgewebe eines Patienten zur Behandlung von Krebs, wobei der Vektor ein adenoviraler Vektor oder ein retroviraler Vektor ist.

17. Rekombinanter Vektor, der pRb2/p130 exprimiert, zur Verwendung in der Inhibierung der Angiogenese in einem Krebsgewebe eines Patienten, zur Behandlung von Krebs, wobei der Vektor ein adenoviraler Vektor oder ein retroviraler Vektor ist.

18. Verwendung oder Vektor nach einem der Ansprüche 3 bis 6, 14, 16 oder 17, wobei der Krebs ein humanes Glioblastom, Melanom, Brustkrebs, Prostatakrebs, Lungenkrebs, ein Endometriom oder ein Magenkarzinom ist.

19. Verwendung eines rekombinanten Vektors, der pRb2/p130 exprimiert, zur Herstellung einer pharmazeutischen Zusammensetzung zur Inhibierung der Angiogenese in Lungenkrebsgewebe eines Patienten, wobei die Dosierung der Zusammensetzung ausreichend ist, um die VEGF-Expression herunterzuregulieren, sodass die Angiogenese in dem Gewebe inhibiert wird und wobei der Vektor ein adenoviraler Vektor oder ein retroviraler Vektor ist.

20. Rekombinanter Vektor, der pRb2/p130 exprimiert, zur Verwendung in der Inhibierung der Angiogenese in Lungenkrebsgewebe eines Patienten, wobei die Dosierung der Zusammensetzung ausreichend ist, um die VEGF-Expression herunterzuregulieren, sodass die Angiogenese in dem Gewebe inhibiert wird und wobei der Vektor ein adenoviraler Vektor oder ein retroviraler Vektor ist.

21. Verwendung eines rekombinanten Vektors, der pRb2/p130 exprimiert, zur Herstellung einer pharmazeutischen Zusammensetzung zur Inhibierung der Angiogenese in einem Glioblastomgewebe eines Patienten, wobei die Dosierung der Zusammensetzung ausreichend ist, um die VEGF-Expression herunterzuregulieren, sodass die Angiogenese in dem Gewebe inhibiert wird und wobei der Vektor ein adenoviraler Vektor oder ein retroviraler Vektor ist.

22. Rekombinanter Vektor, der pRb2/p130 exprimiert, zur Verwendung in der Inhibierung der Angiogenese in einem Glioblastomgewebe eines Patienten, wobei die Dosierung der Zusammensetzung ausreichend ist, um die VEGF-Expression herunterzuregulieren, sodass die Angiogenese in dem Gewebe inhibiert wird und wobei der Vektor ein adenoviraler Vektor oder ein retroviraler Vektor ist.

23. Verwendung eines rekombinanten Vektors, der pRb2/p130 exprimiert, zur Herstellung einer pharmazeutischen Zusammensetzung zur Inhibierung der Angiogenese in einem Prostatakrebsgewebe eines Patienten, wobei die Dosierung der Zusammensetzung ausreichend ist, um die VEGF-Expression herunterzuregulieren, sodass die Angiogenese in dem Gewebe inhibiert wird und wobei der Vektor ein adenoviraler Vektor oder ein retroviraler Vektor ist.

24. Rekombinanter Vektor, der pRb2/p130 exprimiert, zur Verwendung in der Inhibierung der Angiogenese in Prostatakrebsgewebe eines Patienten, wobei die Dosierung der Zusammensetzung ausreichend ist, um die VEGF-Expression herunterzuregulieren, sodass die Angiogenese in dem Gewebe inhibiert wird und wobei der Vektor ein adenoviraler Vektor oder ein retroviraler Vektor ist.

25. Verwendung eines rekombinanten Vektors, der pRb2/p130 exprimiert, zur Herstellung einer pharmazeutischen Zusammensetzung für die Behandlung von rheumatoider Arthritis in einem Patienten, wobei die Dosierung der Zusammensetzung ausreichend ist, um die VEGF-Expression in synovialem Gewebe herunterzuregulieren und die Angiogenese in dem synovialen Gewebe zu inhibieren und wobei der Vektor ein adenoviraler Vektor oder ein retroviraler Vektor ist.

26. Rekombinanter Vektor, der pRb2/p130 exprimiert, zur Verwendung in der Behandlung von rheumatoider Arthritis in einem Patienten, wobei die Dosierung der Zusammensetzung ausreichend ist, um die VEGF-Expression in synovialem Gewebe herunterzuregulieren und die Angiogenese in dem synovialen Gewebe zu inhibieren und wobei der Vektor ein adenoviraler Vektor oder ein retroviraler Vektor ist.

27. Verwendung eines rekombinanten Vektors, der pRb2/p130 exprimiert, zur Herstellung einer pharmazeutischen Zusammensetzung zur Behandlung von diabetischer Retinopathie in einem Patienten, wobei die Dosierung der Zusammensetzung ausreichend ist, um die VEGF-Expression in der Netzhaut herunterzuregulieren und die Angiogenese in der Netzhaut zu inhibieren und wobei der Vektor ein adenoviraler Vektor oder ein retroviraler Vektor ist.

28. Rekombinanter Vektor, der pRb2/p130 exprimiert, zur Verwendung in der Behandlung von diabetischer Retinopathie in einem Patienten, wobei die Dosierung der Zusammensetzung ausreichend ist, um die VEGF Expression in der Netzhaut herunterzuregulieren und die Angiogenese in der Netzhaut zu inhibieren und wobei der Vektor ein adenoviraler Vektor oder ein retroviraler Vektor ist.

29. Verwendung eines rekombinanten Vektors, der pRb2/p130 exprimiert, zur Herstellung einer pharmazeutischen Zusammensetzung zur Behandlung von Aderhaut-Gefäßneubildung in einem Patienten, wobei die Dosierung der Zusammensetzung ausreichend ist, um die VEGF-Expression in dem Gewebe herunterzuregulieren und die Angiogenese in der Netzhaut zu inhibieren und wobei der Vektor ein adenoviraler Vektor oder ein retroviraler Vektor ist.

30. Rekombinanter Vektor, der pRb2/p130 exprimiert, zur Verwendung in der Behandlung von Aderhaut-Gefäßneubildung in einem Patienten, wobei die Dosierung der Zusammensetzung ausreichend ist, um die VEGF-Expression in dem Gewebe herunterzuregulieren und die Angiogenese in der Netzhaut zu inhibieren und wobei der Vektor ein adenoviraler Vektor oder ein retroviraler Vektor ist.

## Revendications

1. Utilisation d'un vecteur exprimant pRb2/p130 pour la préparation d'une composition pharmaceutique pour l'inhibition de l'angiogénèse dans un tissu cible chez un patient ayant besoin de ladite inhibition.

2. Vecteur exprimant pRb2/p130 pour une utilisation dans l'inhibition de l'angiogénèse dans un tissu cible.

3. Utilisation selon la revendication 1 ou vecteur selon la revendication 2, dans laquelle/lequel le tissu cible est un tissu rétinien, un épithélium pigmentaire rétinien, un tissu synovial, une tumeur ou un cancer.

4. Utilisation selon la revendication 1 ou 3 ou vecteur selon la revendication 2 ou 3, dans laquelle/lequel le vecteur est un vecteur rétroviral.

5. Utilisation selon la revendication 1 ou 3 ou vecteur selon la revendication 2 ou 3, dans laquelle/lequel le vecteur est un vecteur adénoviral.

6. Utilisation selon l'une quelconque des revendications 1 et 3 à 5 ou vecteur selon l'une quelconque des revendications 2 à 5, dans laquelle/lequel ladite expression de pRb2/p130 dans le tissu cible entraîne la régulation à la baisse du VEGF dans la zone cible.

7. Utilisation d'un vecteur viral recombinant exprimant pRb2/p130 ou un fragment de celui-ci pour la préparation d'une composition pharmaceutique pour la modulation spécifique de l'expression d'un gène d'intérêt dans une ou plusieurs cellules, dans laquelle le gène code pour une protéine, dont l'expression est associée à l'angiogénèse chez un patient.

8. Vecteur viral recombinant exprimant pRb2/p130 ou un fragment de celui-ci pour une utilisation dans la modulation spécifique de l'expression d'un gène d'intérêt dans une ou plusieurs cellules, dans lequel le gène code pour une protéine, dont l'expression est associée à l'angiogénèse.

9. Utilisation selon la revendication 7 ou vecteur viral recombinant selon la revendication 8, dans laquelle/lequel le gène d'intérêt est le VEGF.

10. Utilisation ou vecteur viral recombinant selon la revendication 9, dans laquelle/lequel pRb2/p130 ou un fragment de celui-ci interfère avec la régulation du promoteur dudit VEGF.

11. Utilisation ou vecteur viral recombinant selon la revendication 9, dans laquelle/lequel pRb2/p130 ou un fragment de celui-ci interfère avec l'expression de l'ARNm dudit VEGF.

12. Utilisation ou vecteur viral recombinant selon la revendication 9, dans laquelle/lequel pRb2/p130 ou un fragment de celui-ci interfère avec l'expression protéique dudit VEGF.

13. Utilisation selon l'une quelconque des revendications 7 et 9 à 12 ou vecteur viral recombinant selon l'une quelconque des revendications 8 à 12, dans laquelle/lequel lesdites cellules sont des cellules d'une tumeur humaine.

14. Utilisation selon l'une quelconque des revendications 7 et 9 à 12 ou vecteur viral recombinant selon l'une quelconque des revendications 8 à 12, dans laquelle/lequel lesdites cellules sont des cellules d'un cancer humain.

15. Utilisation selon l'une quelconque des revendications 7 et 9 à 12 ou vecteur viral recombinant selon l'une quelconque des revendications 8 à 12, dans laquelle/lequel lesdites cellules sont des cellules d'une rétine, d'un épithélium pigmentaire rétinien, des leucocytes synoviaux ou des neutrophiles synoviaux.

16. Utilisation d'un vecteur recombinant exprimant pRb2/p130 pour la préparation d'une composition pharmaceutique pour l'inhibition de l'angiogénèse dans un tissu cancéreux d'un patient pour traiter le cancer, dans laquelle le vecteur est un vecteur adénoviral ou un vecteur rétroviral.

17. Vecteur recombinant exprimant pRb2/p130 pour une utilisation dans l'inhibition de l'angiogénèse dans un tissu cancéreux d'un patient pour traiter le cancer, dans lequel le vecteur est un vecteur adénoviral ou un vecteur rétroviral.

18. Utilisation ou vecteur selon l'une quelconque des revendications 3 à 6, 14, 16 ou 17, dans laquelle/lequel le cancer est un glioblastome, un mélanome, un cancer du sein, un cancer de la prostate, un cancer du poumon, un cancer de l'endomètre ou un carcinome de l'estomac humain.

19. Utilisation d'un vecteur recombinant exprimant pRb2/p130 pour la préparation d'une composition pharmaceutique pour l'inhibition de l'angiogénèse dans un tissu de cancer du poumon d'un patient, dans laquelle le dosage de ladite composition est suffisant pour réguler à la baisse l'expression du VEGF de façon à inhiber l'angiogénèse dans ledit tissu, et le vecteur est un vecteur adénoviral ou un vecteur rétroviral.

20. Vecteur recombinant exprimant pRb2/p130 pour une utilisation dans l'inhibition de l'angiogénèse dans un tissu de cancer du poumon d'un patient, dans lequel le dosage de ladite composition est suffisant pour réguler à la baisse l'expression du VEGF de façon à inhiber l'angiogénèse dans ledit tissu, et le vecteur est un vecteur adénoviral ou un vecteur rétroviral.

21. Utilisation d'un vecteur recombinant exprimant pRb2/p130 pour la préparation d'une composition pharmaceutique pour l'inhibition de l'angiogenèse dans un tissu de glioblastome d'un patient, dans laquelle le dosage de ladite composition est suffisant pour réguler à la baisse l'expression du VEGF de façon à inhiber l'angiogénèse dans ledit tissu, et le vecteur est un vecteur adénoviral ou un vecteur rétroviral.

22. Vecteur recombinant exprimant pRb2/p130 pour une utilisation dans l'inhibition de l'angiogénèse dans un tissu de glioblastome d'un patient, dans lequel le dosage de ladite composition est suffisant pour réguler à la baisse l'expression du VEGF de façon à inhiber l'angiogénèse dans ledit tissu, et le vecteur est un vecteur adénoviral ou un vecteur rétroviral.

23. Utilisation d'un vecteur recombinant exprimant pRb2/p130 pour la préparation d'une composition pharmaceutique pour l'inhibition de l'angiogénèse dans un tissu de cancer de la prostate d'un patient, dans laquelle le dosage de ladite composition est suffisant pour réguler à la baisse l'expression du VEGF de façon à inhiber l'angiogénèse dans ledit tissu, et le vecteur est un vecteur adénoviral ou un vecteur rétroviral.

24. Vecteur recombinant exprimant pRb2/p130 pour une utilisation dans l'inhibition de l'angiogénèse dans un tissu de cancer de la prostate d'un patient, dans lequel le dosage de ladite composition est suffisant pour réguler à la baisse l'expression du VEGF de façon à inhiber l'angiogénèse dans ledit tissu, et le vecteur est un vecteur adénoviral ou un vecteur rétroviral.

25. Utilisation d'un vecteur recombinant exprimant pRb2/p130 pour la préparation d'une composition pharmaceutique pour le traitement de la polyarthrite rhumatoïde chez un patient, dans laquelle le dosage de ladite composition est suffisant pour réguler à la baisse l'expression du VEGF dans le tissu synovial et inhiber l'angiogénèse dans ledit tissu synovial, et le vecteur est un vecteur adénoviral ou un vecteur rétroviral.

26. Vecteur recombinant exprimant pRb2/p130 pour une utilisation dans le traitement de la polyarthrite rhumatoïde chez un patient, dans lequel le dosage de ladite composition est suffisant pour réguler à la baisse l'expression du VEGF dans le tissu synovial et inhiber l'angiogénèse dans ledit tissu synovial, et le vecteur est un vecteur adénoviral ou un vecteur rétroviral.

27. Utilisation d'un vecteur recombinant exprimant pRb2/p130 pour la préparation d'une composition pharmaceutique pour le traitement de la rétinopathie diabètique chez un patient, dans laquelle le dosage de ladite composition est suffisant pour réguler à la baisse l'expression du VEGF dans la rétine et inhiber l'angiogénèse dans la rétine, et le vecteur est un vecteur adénoviral ou un vecteur rétroviral.

28. Vecteur recombinant exprimant pRb2/p130 pour une utilisation dans le traitement de la rétinopathie diabètique chez un patient, dans lequel le dosage de ladite composition est suffisant pour réguler à la baisse l'expression du VEGF dans la rétine et inhiber l'angiogénèse dans la rétine, et le vecteur est un vecteur adénoviral ou un vecteur rétroviral.

29. Utilisation d'un vecteur recombinant exprimant pRb2/p130 pour la préparation d'une composition pharmaceutique pour le traitement de la néovascularisation choroïdienne chez un patient, dans laquelle le dosage de ladite composition est suffisant pour réguler à la baisse l'expression du VEGF dans ledit tissu et inhiber l'angiogénèse dans la rétine, et le vecteur est un vecteur adénoviral ou un vecteur rétroviral.

30. Vecteur recombinant exprimant pRb2/p130 pour une utilisation dans le traitement de la néovascularisation choroïdienne chez un patient, dans lequel le dosage de ladite composition est suffisant pour réguler à la baisse l'expression du VEGF dans ledit tissu et inhiber l'angiogénèse dans la rétine, et le vecteur est un vecteur adénoviral ou un vecteur rétroviral.
